# EUROPÄISCHE PATENTSCHRIFT

(11) **EP 2 009 425 B1**
(45) Veröffentlichungstag und Bekanntmachung des Hinweises auf die Patenterteilung: **12.01.2011**
(21) Anmeldenummer: 08010934.1
(22) Anmeldetag: 17.06.2008
(51) Int. Cl.: G01N 21/898

(54) **Vorrichtung und Verfahren zum Scannen von Massivhölzern**
Apparatus and method for scanning of solid wood
Dispositif et procédé pour l'inspection du bois massif

(30) Priorität: 25.06.2007 DE 102007030865
(43) Veröffentlichungstag der Anmeldung: 31.12.2008
(73) Patentinhaber: GreCon Dimter Holzoptimierung Süd GmbH & Co. KG, 89257 Illertissen (DE)
(72) Erfinder: Fahrenschon, Walter, 89264 Weissenhorn (DE)
(74) Vertreter: Jackisch-Kohl, Anna-Katharina

(56) Entgegenhaltungen:
- EP-A- 0 234 492
- EP-A- 1 219 955
- EP-A- 1 630 519
- WO-A-85/00657
- WO-A-98/07023
- WO-A-2004/083778
- DE-A1- 19 716 468
- US-A- 5 257 101
- US-B1- 6 756 789

## Beschreibung

Die Erfindung betrifft eine Vorrichtung zum Scannen von Massivhölzern nach dem Oberbegriff des Anspruches 1 sowie ein Verfahren zum Scannen von Massivhölzern nach dem Oberbegriff des Anspruches 12.

In der Holzbranche werden Scannereinheiten zur Erkennung von Holzfehlern und zur Bestimmung der Holzqualität eingesetzt. Die Scannereinheit mit Beleuchtung ist stationär angeordnet. Die Massivhölzer werden einzeln mittels entsprechender Transporteinrichtungen durch die Scannereinheit transportiert. Brettanfang und Brettende werden mittels einer geeigneten Sensorik erfasst und die Längeninformation über das Massivholz mittels Impulsgeber ermittelt. Die so ermittelten Brettdaten werden zu einer Schnittliste optimiert, nach der das Massivholz in einer nachfolgenden Sägestation in Form einer Durchlaufsäge gesägt wird. Bei anderen Anwendungen werden die Massivhölzer einer Sortierstation zugeführt und entsprechend der ermittelten Qualitätsvorgaben sortiert.

Außer den Durchlaufsägen, bei denen die Massivhölzer mittels Vorschubrollen, Ketten, Zahnriemen positioniert und transportiert werden, sind auch Schiebersägen bekannt, bei denen das Massivholz mittels eines Schiebers und eines Linearmoduls positioniert und transportiert wird.

Es ist bekannt (WO 98/07023A), das Massivholz in eine Scannvorrichtung einzugeben und die Ober- und die Unterseite des Massivholzes durch jeweils eine Scannereinheit abzuscannen. Die beiden Scannereinheiten werden hierzu längs des Massivholzes verfahren. Nach dem Scannvorgang wird das Massivholz der Scannvorrichtung entnommen und der weiteren Ver/Bearbeitung zugeführt.

Es ist weiter bekannt (US-A-5 257 101), das Massivholz mittels einer Scannereinheit abzuscannen, die längs des Massivholzes verfahren wird. Während des Scannvorganges wird das Massivholz um seine Längsachse mit Hilfe von Rollen gedreht. Nach dem Scannvorgang wird das Massivholz der Scannvorrichtung entnommen und weitertransportiert.

Schließlich ist es bekannt (EP-A-0 234 492), ein Massivholz während des Scannens durch ein Portal zu transportieren, an dem eine Scannereinheit befestigt ist.

Der Erfindung liegt die Aufgabe zugrunde, die gattungsgemäße Vorrichtung und das gattungsgemäße Verfahren so auszubilden, dass die Massivhölzer bei einfacher konstruktiver Gestaltung der Vorrichtung zuverlässig gescannt werden können.

Diese Aufgabe wird bei der gattungsgemäßen Vorrichtung erfindungsgemäß mit den kennzeichnenden Merkmalen des Anspruches 1 und beim gattungsgemäßen Verfahren erfindungsgemäß mit den kennzeichnenden Merkmalen des Anspruches 12 gelöst.

Mit der erfindungsgemäßen Vorrichtung wird die Scannereinheit zusammen mit dem Schieber, mit dem das Massivholz einer nachfolgenden Sägestation zugeführt wird, am Massivholz entlang geführt, das hierbei gescannt wird. Da der Schieber ohnehin zum Transport des Massivholzes in die Sägestation oder in die Sortierstation verfahren werden muss, kann der vom Schieber auszuführende Hub zugleich zum Scannen des Massivholzes genutzt werden.

Vorteilhaft weist die Scannereinheit zum Scannen des Massivholzes wenigstens eine Kamera auf. Mit ihr kann das Massivholz einwandfrei gescannt werden.

Damit der Scannvorgang rasch durchgeführt werden kann, weist die Scannereinheit über den Umfang des Massivholzes verteilt mehrere Kameras auf. Auf diese Weises können mit den Kameras alle Seiten des Massivholzes bei einer Hubbewegung des Schiebers erfasst werden.

Es ist vorteilhaft möglich, für jede Seite des Massivholzes jeweils eine Kamera vorzusehen.

Ebenso ist aber auch möglich, dass eine Kamera zwei Seiten des Massivholzes gleichzeitig erfasst. Bei einem viereckigen Massivholz sind somit nur zwei Kameras erforderlich, die jeweils zwei Seiten des Massivholzes erfassen. Auch in diesem Falle ist nur ein Hub des Schiebers notwendig, um alle Seiten des Massivholzes über dessen Länge mit den beiden Kameras zu erfassen.

Bei einer anderen vorteilhaften Ausbildung hat die Scannereinheit eine Kamera und einen mit ihr zusammenwirkenden Spiegel. Die Kamera und der Spiegel sind so einander zugeordnet, dass die Kamera selbst zwei Seiten des Massivholzes und über den Spiegel die beiden anderen Seiten des viereckigen Querschnitts aufweisenden Massivholzes erfassen kann. Somit können mit einem einzigen Hub des Schiebers alle Seiten des Massivholzes über dessen Länge gescannt werden.

Damit die Kamera optimal eingestellt werden kann, ist sie vorteilhaft einstellbar am Schieber vorgesehen.

Auch der Spiegel ist vorteilhaft einstellbar am Schieber angeordnet, so dass auch er auf das jeweils zu scannende Massivholz eingestellt werden kann.

Während des Scannvorganges ruht das Massivholz vorteilhaft auf Auflagen.

Bei einer anderen Ausführungsform wird das Masssivholz zwischen zwei Drehvorrichtungen aufgenommen. Hierbei ist vorteilhaft wenigstens eine Drehvorrichtung antreibbar, um das Massivholz um seine Längsachse drehen zu können.

Zumindest die eine Drehvorrichtung ist in Verfahrrichtung der Scannereinheit in Richtung auf die andere Drehvorrichtung verstellbar. Dadurch lassen sich unterschiedlich lange Massivhölzer problemlos zwischen den beiden Drehvorrichtungen aufnehmen.

Vorteilhaft scannt die Scannereinheit das Massivholz beim Rückhub von der Sägestation.

Bei einer anderen vorteilhaften Ausgestaltung wird das ruhende Massivholz sowohl beim Vorwärts- als auch beim Rückhub der Scannereinheit gescannt. Während der beiden Scannvorgänge ruht das Massivholz. Zwischen den beiden Hüben wird es um seine Längsachse gedreht.

Weitere Merkmale der Erfindung ergeben sich aus den weiteren Ansprüchen, der Beschreibung und den Zeichnungen.

Die Erfindung wird anhand dreier in den Zeichnungen dargestellter Ausführungsformen näher erläutert. Es zeigen
- Fig. 1: in perspektivischer und vereinfachter Darstellung eine erste Ausführungsform einer erfindungsgemäßen Vorrichtung,
- Fig. 2 und 3: in Darstellungen entsprechend Fig. 1 jeweils eine weitere Ausführungsform einer erfindungsgemäßen Vorrichtung.

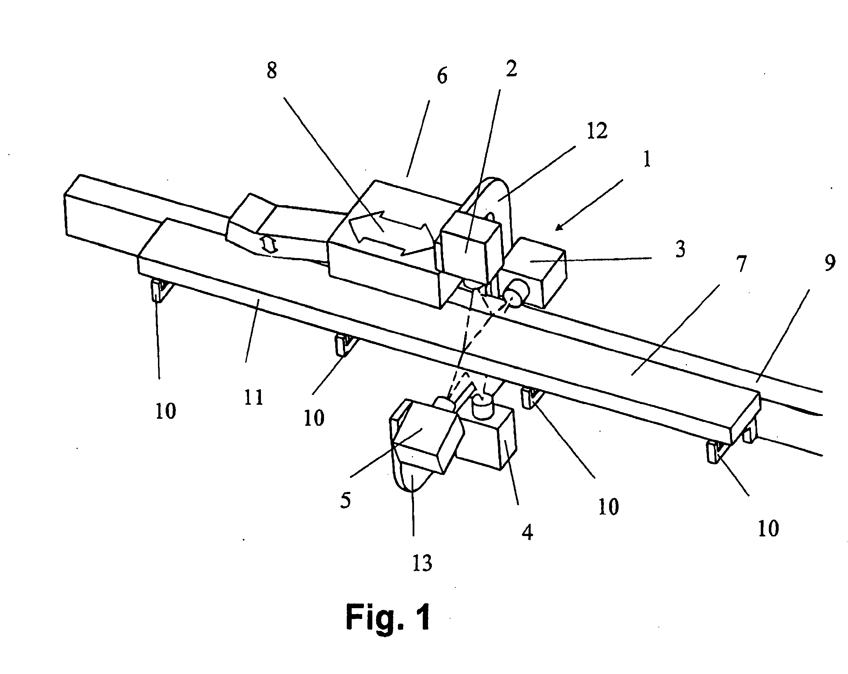

Die Vorrichtung gemäß Fig. 1, mit der Holzfehler erkannt und Holzqualitäten bestimmt werden, hat eine Scannereinheit 1, die vier Kameras 2 bis 5 mit (nicht dargestellter) Beleuchtung aufweist. Die Scannereinheit 1 ist an einem Schieber 6 vorgesehen, der in Transportrichtung des Massivholzes 7 in Pfeilrichtung 8 verfahrbar ist. Der Schieber 6 ist längs einer Führung 9 verfahrbar, von der quer schmale Auflagen 10 abstehen, auf denen das zu scannende Massivholz 7 aufliegt. Die Auflagen 10 sind mit Abstand hintereinander angeordnet.

Die Kameras 2 bis 5 sind an wenigstens einem Träger 12 gehalten, dessen einer Arm 13 sich unterhalb des Massivholzes 7 bis über dessen Schmalseite 11 hinaus erstreckt. Die Kameras 2 bis 5 können vorteilhaft gegenüber dem Träger 12 um jeweils eine in Verschieberichtung 8 liegende Achse verstellt werden. Die Kameras 2 bis 5 lassen sich dadurch optimal auf das zu scannende Massivholz einstellen.

Das Massivholz 7 befindet sich in der in Fig. 1 dargestellten Lage in der Beschickstation zum nachfolgenden Sägen. Mit dem Schieber 6 ist das vorige Massivholz 7 bereits zur Säge transportiert worden. Beim Rückhub fährt der Schieber 6 an dem in der Beschickstation befindlichen Massivholz entlang. Die Kameras 2 bis 5 nehmen dabei die Brettdaten auf, die anschließend zu einer Schnittliste optimiert werden. Die Kameras 2 bis 5 sind so am Träger 12 eingestellt, dass sie alle vier Seiten des Massivholzes erfassen können. Im Ausführungsbeispiel ist das Massivholz 7 als flaches Brett ausgebildet. Die Kameras 2, 4 befinden sich mit Abstand oberhalb und unterhalb des Massivholzes 7 und erfassen beim Rückhub des Schlittens 6 dessen Ober- und Unterseite. Die beiden Kameras 3, 5 befinden sich mit Abstand neben dem Massivholz 7 und erfassen dessen beide Schmalseiten. Beim Rückhub erkennen die Kameras 2 bis 5 Fehler. Außerdem wird mit ihnen die Holzgüte bestimmt. Auch die Länge des Massivholzes 7 kann mit der Scannereinheit 1 bestimmt werden. Diese Informationen bilden die Brettdaten, die zu einer Schnittliste optimiert werden. Sie wird in bekannter Weise der nachfolgenden Sägeneinheit zugeführt, die anhand der Daten der Schnittliste das Massivholz sägt. Sobald der Schieber 6 beim Rückhub am Massivholz 7 vorbei gefahren ist, wird seine Verschieberichtung umgekehrt. Er nimmt nunmehr das zuvor gescannte Massivholz 7 mit und führt es der Säge zu. In der Zwischenzeit wird in der Beschickstation das nächste Massivholz 7 auf den Auflagen 10 abgelegt, das beim Rückhub des Schlittens 6 mit dessen Scannereinheit 1 in der beschriebenen Weise erfasst wird. Auf diese Weise werden nacheinander die der Säge zuzuführenden Massivhölzer jeweils beim Rückhub des Schiebers 6 mittels der Scannereinheit 1 erfasst.

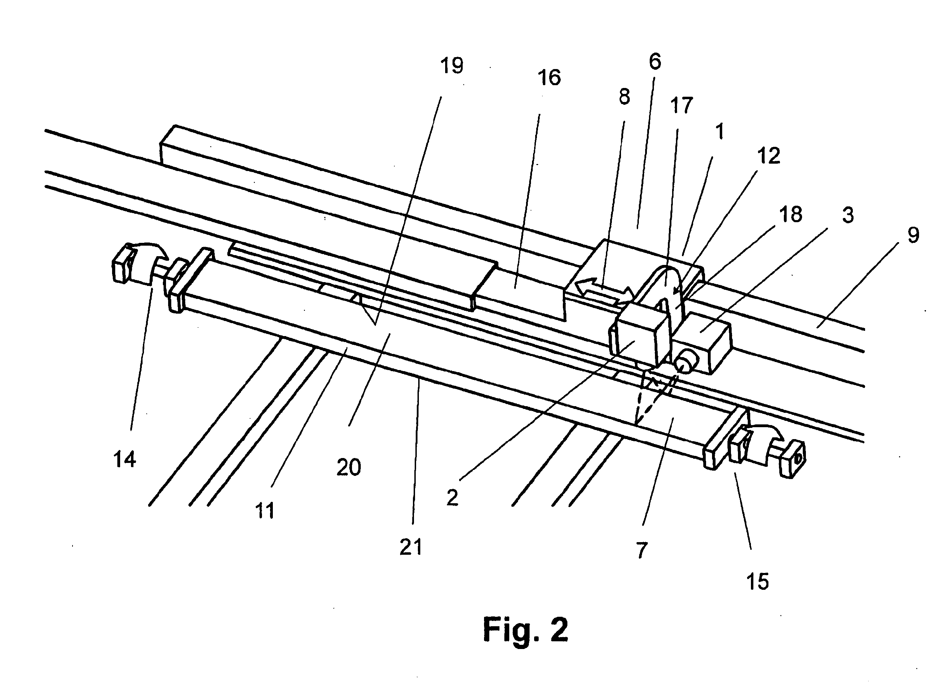

Bei der Ausführungsform gemäß Fig. 2 liegt das brettförmige Massivholz 7 in einer Bereitstellposition vor der Beschickposition. Das Massivholz 7 liegt entsprechend der vorigen Ausführungsform parallel zur Verschieberichtung 8 des Schiebers 6. Das Massivholz 7 ist an seinen beiden Enden durch jeweils eine Drehvorrichtung 14, 15 gehalten, deren Drehachse parallel zur Verschieberichtung 8 des Schiebers 6 verläuft. Der Schieber 6 sitzt auf dem freien Ende eines Armes 16 und wird auf der Führung 9 abgestützt. Der Schieber 6 hat den Träger 12, an dem zwei Kameras 2, 3 gehalten sind. Entsprechend der vorigen Ausführungsform hat der Träger L-Form, wobei die Kamera 2 am freien Ende des einen Armes 17 und die Kamera 3 am freien Ende des anderen Armes 18 des L-förmigen Trägers angeordnet ist. Der Trägerarm 17 erstreckt sich quer zur Verschieberichtung 8 bis über das Massivholz 7. Dadurch kann die Kamera 2 die ihr zugewandte Oberseite 20 des Massivholzes 7 erfassen. Die am nach unten gerichteten Trägerarm 18 gelagerte Kamera 3 ist so angeordnet, dass sie die dem Schieber 6 zugewandte Schmalseite 19 des Massivholzes 7 erfassen kann. Die beiden Kameras 2, 3 oder auch nur eine von ihnen ist vorteilhaft um eine in Verschieberichtung 8 liegende Achse schwenkbar am Träger 12 gelagert, so dass die Kameras 2, 3 optimal in Bezug auf das zu prüfende Massivholz 7 eingestellt werden können.

Der Schieber 6 befindet sich zu Beginn des Scannvorganges im Bereich hinter dem Massivholz 7. Aus dieser Stellung wird der Schieber 6 mit der Scannereinheit 1 in Fig. 2 nach rechts verfahren, wobei die beiden Kameras 2, 3 die Oberseite 20 und die Schmalseite 19 des Massivholzes 7 erfassen. Während dieses Hubs des Schlittens 6 wird zeitgleich in der Sägestation das vorhergehende Massivholz gesägt. Sobald die Scannereinheit 1 die Schmalseite 19 und die Oberseite 20 des Massivholzes 7 erfasst hat, wird es mittels der beiden Drehvorrichtungen 14, 15 um 180° um seine Längsachse gedreht. Nunmehr wird der Schieber 8 mit Scannereinheit 1 zurückgefahren. Die Kameras 2, 3 erfassen hierbei die nun oben liegende Unterseite 21 und die Schmalseite 11 des Massivholzes 7. Die auf Grund des Vor- und des Rückhubes aufgenommenen Brettdaten werden zur Schnittliste optimiert, die der nachfolgenden Sägestation übergeben wird. Mit dem Schieber 6 wird anschließend das Massivholz 7 zur Sägestation transportiert. Nach der Übergabe des Brettes wird vorteilhaft das nächste Massivholz 7 in die Drehvorrichtungen 14, 15 eingespannt. Wenigstens eine der beiden Drehvorrichtungen 14, 15 ist in Verschieberichtung 8 verstellbar, so dass mit den beiden Drehvorrichtungen 14, 15 unterschiedlich lange Massivhölzer 7 eingespannt werden können.

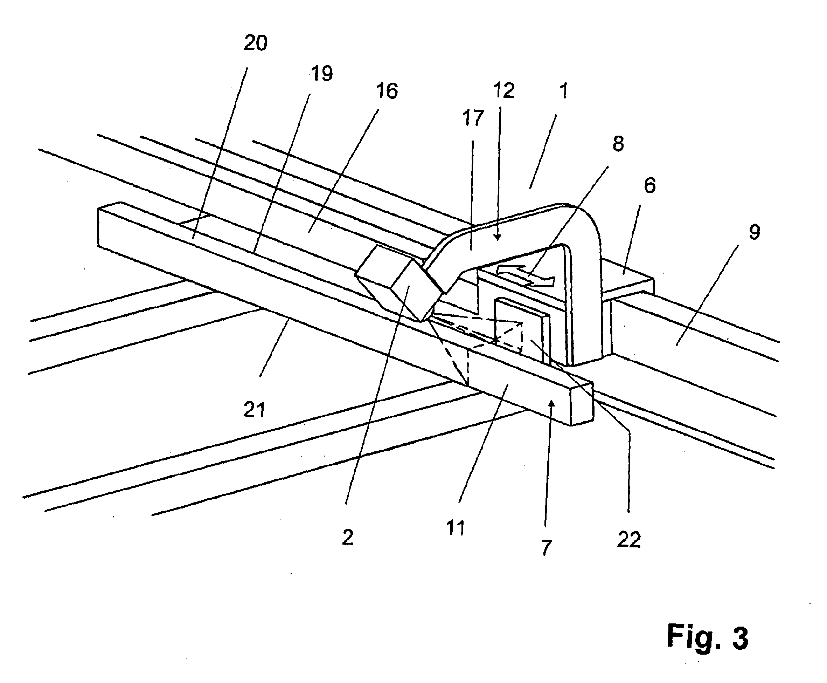

Bei der Ausführungsform gemäß Fig. 3 befindet sich das Massivholz 7 in einer Bereitstellposition vor der Beschickposition. Im Unterschied zu den vorigen Ausführungsbeispielen ist das Massivholz 7 nicht brettförmig, sondern balkenförmig ausgebildet. Der Schieber 6 ist mit der Scannereinheit 1 versehen und auf der Führung 9 abgestützt. Der Schieber 6 wird mit dem Arm 16 in Verschieberichtung 8 verschoben. Die Scannereinheit 1 hat den L-förmigen Träger 12. Am freien Enden des oberen Armes 17 befindet sich die Kamera 2, die sich im Bereich oberhalb und seitlich neben dem Massivholz 7 befindet. Die Kamera 2 ist so eingestellt, dass sie die vom Schieber abgewandte Seite 11 sowie die Oberseite 20 des Massivholzes 7 erfassen kann. Die Kamera 2 ist zu diesem Zweck schräg angeordnet, beispielsweise unter einem Winkel von 45° zur Vertikalen. Vorteilhaft ist es, wenn der Neigungswinkel der Kamera 2 eingestellt werden kann. In diesem Falle ist die Kamera 2 am Träger 12 schwenkbar gelagert, so das sie optimal eingestellt werden kann.

Am Schieber 6 ist außerdem wenigstens ein Spiegel 22 vorgesehen, der so angeordnet ist, dass die Kamera 2 über den Spiegel 22 auch die Seite 19 und die Unterseite 21 des Massivholzes 7 erfassen kann. Der Spiegel 22 ist vorteilhaft höhenverstellbar und/oder in der Neigung einstellbar am Schieber 6 angeordnet.

Mit dieser Vorrichtung erfolgt der Scannvorgang entweder beim Vorfahren des Schiebers 6 oder beim Rückhub. Mit der einzigen Kamera 2 können in Verbindung mit dem Spiegel 22 alle vier Seiten des Massivholzes 7 erfasst werden. Hierbei werden die Brettdaten aufgenommen und zur Schnittliste optimiert, anhand der das Massivholz 7 in der nachfolgenden Sägestation gesägt werden kann.

Die bei den verschiedenen Vorrichtungen vorgesehenen Kameras können selbstverständlich in anderer Zahl und Anordnung am Schieber 6 vorgesehen, als in den Ausführungsbeispielen gemäß den Fig. 1 bis 3 dargestellt und erläutert worden ist. Da die Scannereinheit 1 mit dem Schieber 6 mitfährt, ergibt sich eine einfache und dennoch zuverlässige Erkennung von Holzfehlern und Holzqualitäten.

## Patentansprüche

1. Vorrichtung zum Scannen von Massivhölzern (7) mit wenigstens einer Scannereinheit (1), mit der wenigstens eine Seite (11, 19 bis 21) des Massivholzes (7) erfasst wird und die längs des zu scannenden Massivholzes (7) verfahrbar ist,
**dadurch gekennzeichnet, dass** die Scannereinheit (1) mit einem Schieber (6) verbunden ist, der zum Transport des Massivholzes (7) in dessen Längsrichtung längs einer Führung (9) in der Vorrichtung verschiebbar ist.

2. Vorrichtung nach Anspruch 1,
**dadurch gekennzeichnet, dass** die Scannereinheit (1) wenigstens eine Kamera (2 bis 5) aufweist, mit der das Massivholz (7) scannbar ist.

3. Vorrichtung nach Anspruch 2,
**dadurch gekennzeichnet, dass** die Scannereinheit (1) über den Umfang des Massivholzes (7) verteilt mehrere Kameras (2 bis 5) aufweist.

4. Vorrichtung nach Anspruch 2 oder 3,
**dadurch gekennzeichnet, dass** bei einem Vierkantholz als Massivholz (7) für jede Seite des Massivholzes (7) jeweils eine Kamera (2 bis 5) vorgesehen ist.

5. Vorrichtung nach Anspruch 2 oder 3,
**dadurch gekennzeichnet, dass** bei einem Vierkantholz als Massivholz (7) für zwei Seiten (19, 20; 11, 21) des Massivholzes (7) jeweils eine Kamera (2, 3) vorgesehen ist.

6. Vorrichtung nach Anspruch 2 oder 3,
**dadurch gekennzeichnet, dass** bei einem Vierkantholz als Massivholz (7) zur Erfassung aller vier Seiten (11, 19 bis 21) des Massivholzes (7) eine Kamera (2) und mindestens ein mit ihr zusammenwirkender Spiegel (22) vorgesehen sind.

7. Vorrichtung nach Anspruch 6,
**dadurch gekennzeichnet, dass** die Kamera (2) zwei Seiten (11, 20) und der Spiegel (22) die beiden anderen Seiten (19, 21) des Massivholzes (7) erfasst.

8. Vorrichtung nach einem der Ansprüche 4 bis 7,
**dadurch gekennzeichnet, dass** die Kamera (2 bis 5) und/oder der Spiegel (22) einstellbar am Schieber (6) vorgesehen ist.

9. Vorrichtung nach einem der Ansprüche 1 bis 8,
**dadurch gekennzeichnet, dass** das Massivholz (7) auf Auflagen (10) aufliegt.

10. Vorrichtung nach einem der Ansprüche 1 bis 8,
**dadurch gekennzeichnet, dass** das Massivholz (7) zwischen zwei Drehvorrichtungen (14, 15) aufgenommen ist.

11. Vorrichtung nach Anspruch 10,
**dadurch gekennzeichnet, dass** wenigstens eine Drehvorrichtung (14, 15) antreibbar und/oder in Verfahrrichtung (8) der Scannereinheit (1) verstellbar ist.

12. Verfahren zum Scannen von Massivhölzern (7), insbesondere mit einer Vorrichtung nach einem der Ansprüche 1 bis 11, bei dem das Massivholz (7) an wenigstens einer Seite (11, 19 bis 21) über seine Länge hinsichtlich Holzfehler/Holzqualität gescannt wird, indem die Scannereinheit (1) beim Scannvorgang wenigstens einmal längs des Massivholzes (7) verfahren wird,
**dadurch gekennzeichnet, dass** die Scannereinheit (1) längs des Massivholzes (7) mit Hilfe eines Schiebers (6) verschoben wird, der längs einer Führung (9) der Vorrichtung verfahren wird und mit dem das Massivholz (7) einer nachfolgenden Sägestation oder Sortierstation zugeführt wird.

13. Verfahren nach Anspruch 12,
**dadurch gekennzeichnet, dass** das Massivholz (7) während des Scannvorganges ruht.

14. Verfahren nach Anspruch 12 oder 13,
**dadurch gekennzeichnet, dass** die Scannereinheit (1) beim Rückhub von der Sägestation das Massivholz (7) scannt.

15. Verfahren nach Anspruch 12 oder 13,
**dadurch gekennzeichnet, dass** das ruhende Massivholz (7) beim Vorwärtshub und beim Rückhub der Scannereinheit (1) gescannt wird, wobei das Massivholz (7) während der beiden Hübe ruht, zwischen den Hüben jedoch um seine Längsachse gedreht wird.

## Claims

1. Apparatus for scanning of solid wood (7), with at least one scanning unit (1), with which at least one side (11, 19 to 21) of the solid wood (7) is registered and which can travel along the solid wood (7) to be scanned,
**characterised in that** the scanning unit (1) is connected to a slider (6), which is movable for the transporting of the solid wood (7) in its lengthwise direction along a guide (9) within the apparatus.

2. Apparatus according to claim 1,
**characterised in that** the scanning unit (1) comprises at least one camera (2 to 5), with which the solid wood (7) can be scanned.

3. Apparatus according to claim 2,
**characterised in that** the scanning unit (1) comprises several cameras (2 to 5), distributed about the circumference of the solid wood (7).

4. Apparatus according to claim 2 or 3,
**characterised in that** with a square piece of solid wood (7), a camera (2 to 5) is provided for each side of the solid wood (7), respectively

5. Apparatus according to claim 2 or 3,
**characterised in that** with a square piece of solid wood (7), a camera (2, 3) is assigned to two sides (19, 20; 11,21) of the solid wood (7), respectively

6. Apparatus according to claim 2 or 3,
**characterised in that** with a square piece of solid wood (7), there are provided for detecting all four sides (11, 19 to 21) of the solid wood (7) one camera (2) and at least one mirror (22) cooperating with it are provided.

7. Apparatus according to claim 6,
**characterised in that** the camera (2) detects two sides (11, 20) and the mirror the two other sides (19, 21) of the solid wood (7).

8. Apparatus according to one of the claims 4 to 7,
**characterised in that** the camera (2 to 5) and/or the mirror (22) are adjustably arranged on the pusher (6).

9. Apparatus according to one of the claims 1 to 8,
**characterised in that** the solid wood (7) rests on supports (10).

10. Apparatus according to one of the claims 1 to 8,
**characterised in that** the solid wood (7) is placed between two rotating devices (14, 15).

11. Apparatus according to claim 10,
**characterised in that** at least one rotating device (14, 15) can be driven and/or is adjustable in a travel direction (8) of the scanning unit (1).

12. Method for scanning of solid wood (7), particularly with an apparatus according to one of the claims 1 to 11, in which the solid wood (7) is scanned on at least one side (11, 19 to 21) across its length for detecting flaws quality of the wood by driving the scanning unit (1) at least once across a length of the solid wood (7) during the scanning operation,
**characterised in that** the scanning unit (1) is moved a length the solid wood (7) by a pusher (6), which moves across a length on a guiding (9) along the apparatus and with which the solid wood (7) is conveyed to a subsequent sawing station or sorting station.

13. Method according to claim 12,
**characterised in that** the solid wood (7) is at rest during the scanning operation.

14. Method according to claim 12 or 13,
**characterised in that** the scanning unit (1) scans the solid wood (7) during the return stroke from the sawing station.

15. Method according to claim 12 or 13,
**characterised in that** the stationary solid wood (7) is scanned during the advancing stroke and during the return stroke, wherein the solid wood (7) is at rest during both strokes, however is rotating about its longitudinal axis between the strokes.

## Revendications

1. Dispositif pour l'inspection du bois massif (7) avec au moins une unité de scanner (1) avec laquelle au moins un côté (11, 19 à 21) est saisi, étant mobile le long du bois massif (7) à scanner,
**caractérisé en ce que** l'unité de scanner (1) est reliée avec une coulisse (6) déplaçable pour le transport du bois massif (7) dans le sens longitudinal de celui-ci le long d'un guidage (9) dans le dispositif.

2. Dispositif selon revendication 1,
**caractérisé en ce que** l'unité de scanner (1) comprend au moins une caméra (2 à 5), avec lequel le bois massif (7) peut être scanné.

3. Dispositif selon revendication 2,
**caractérisé en ce que** l'unité de scanner (1) comprend plusieurs caméras (2 à 5), espacés sur le circonférence du bois massif (7).

4. Dispositif selon revendication 2 ou 3,
**caractérisé en ce que** en cas d'un bois quadrangulaire comme bois massif (7) pour chaque côté du bois massif (7) une caméra (2 à 5) est prévu.

5. Dispositif selon revendication 2 ou 3,
**caractérisé en ce que** en cas d'un bois guardangulaire comme bois massif (7) pour deux côtés (19, 20; 11, 21) du bois massif (7) respectivement une caméra (2, 3) est prévu.

6. Dispositif selon revendication 2 ou 3,
**caractérisé en ce que** en cas d'un bois quadrangulaire comme bois massif (7) pour le enregistrement des tous les quatre côtés (11, 19 à 21) du bois massif (7) une caméra(2) et au moins un miroir (22), coopérer avec celui-ci, sont prévus.

7. Dispositif selon revendication 6,
**caractérisé en ce que** la caméra (2) saisit deux côtés (11, 20) et le miroir (22) les deux autres côtés (19, 21) du bois massif (7).

8. Dispositif selon une des revendications 4 à 7,
**caractérisé en ce que** la caméra (2 à 5) et/ou le miroir (22) sont prévus ajustables au la coulisse (6).

9. Dispositif selon une des revendications 1 à 8,
**caractérisé en ce que** le bois massif (7) est posé sur des appuis (10).

10. Dispositif selon une des revendications 1 à 8,
**caractérisé en ce que** le bois massif (7) est pris entre deux mécanismes de rotation (14, 15).

11. Dispositif selon revendication 10,
**caractérisé en ce que** au moins un mécanisme de rotation (14, 15) peut être entraîné et/ou est déplaçable en direction de marche (8) de l'unité de scanner (1).

12. Procédé pour scanner du bois massif (7), en particulier avec un dispositif selon une des revendications 1 à 11, dans lequel le bois massif (7) est scanné sur au moins un côté (11, 19 à 21) sur sa longueur en vue de défauts du bois / de la qualité du bois, l'unité de scanner (1) se déplaçant lors du processus de scanner au moins une fois sur la longueur du bois massif (7),
**caractérisé en ce que** l'unité de scanner (1) est déplacé le long du bois massif (7) à l'aide d'une coulisse (6), laquelle étant déplacée le long d'un guidage (9) du dispositif et avec laquelle le bois massif (7) est amené à une station de sciage ou de triage.

13. Procédé selon revendication 12,
**caractérisé en ce que** le bois massif (7) reste immobile pendant le processus de scanner.

14. Procédé selon revendication 12 ou 13,
**caractérisé en ce que** l'unité de scanner (1) scanne le bois massif (7) lors de la course de retour de la station de sciage.

15. Procédé selon revendication 12 ou 13,
**caractérisé en ce que** le bois massif (7) en position immobile est scanné lors de la course d'avance et de la course de retour de l'unité de scanner (1), le bois massif (7) étant immobile pendant les deux courses, mais étant toutefois tourné autour de son axe longitudinal entre les courses.
